# EUROPEAN PATENT APPLICATION

(11) **EP 1 982 709 A1**
(43) Date of publication of application: **22.10.2008**
(21) Application number: 07007930.6
(22) Date of filing: 19.04.2007
(51) Int. Cl.: A61K 31/167, A61K 31/57, A61P 11/06

(54) **Use of a composition comprising formoterol and beclomethasone dipropionate for the prevention or treatment of an acute condition of asthma**

(71) Applicant: CHIESI FARMACEUTICI S.p.A., 43100 Parma (IT)
(72) Inventor: Chiesi, Paolo, 43100 Parma (IT); Rondelli, Ivano, 43100 Parma (IT); Acerbi, Daniela, 43100 Parma (IT); Poli, Gianluigi, 43100 Parma (IT)
(74) Representative: Minoja, Fabrizio

(57) **Abstract**

The present invention relates to the use of a composition comprising a fixed combination of
a) formoterol, a pharmaceutically acceptable salt or solvate thereof or a solvate of such a salt; and
b) beclomethasone dipropionate;
for the manufacture of a medicament for use in the prevention or treatment of an acute condition of asthma, intermittent asthma and/or episodes in chronic asthma, more preferably in the rescue treatment of asthma, and in particular in case of acute exacerbations during the maintenance therapy of asthma.

## Description

### FIELD OF THE INVENTION

The present invention relates to the use of a composition comprising a fixed combination of
a) formoterol, a pharmaceutically acceptable salt or solvate thereof or a solvate of such a salt; and
b) beclomethasone dipropionate;
for the manufacture of a medicament for use in the prevention or treatment of an acute condition of asthma, intermittent asthma and/or episodes in chronic asthma, more preferably in the rescue treatment of asthma, and in particular in case of acute exacerbations during the maintenance therapy of asthma.

The invention further relates to a method for prevention or treatment of an acute condition of asthma and/or episodes in chronic asthma by administering by inhalation of a composition comprising the active ingredients (a) and (b) as defined previously.

### BACKGROUND OF THE INVENTION

Asthma is a disease which is becoming more prevalent and is the most common disease of childhood. It can be identified by recurrent wheeze and intermittent air flow limitation. Despite many advances in its understanding, said pathology remains a poorly understood and often poorly treated disease. Previously, contraction of airway smooth muscles has been regarded as the most important feature of asthma. Recently there has been a marked change in the way asthma is managed, deriving from the fact that asthma is recognized as a chronic inflammatory disease. Uncontrolled airway inflammation may lead to mucosal damage and structural changes causing irreversible narrowing of the airways and fibrosis of the lung tissue. Therapy should therefore be aimed at controlling symptoms so that normal life is possible and at the same time provide basis for treating the underlying inflammation.

The acute asthma symptoms can be relieved by first generation beta-2 adrenoceptor agonists such as salbutamol, fenoterol and terbutalin (short-acting beta-2 agonists) or second generation ones such as formoterol and salmeterol (long-acting beta-2 agonists) which overcome the disadvantage of the short duration of action particularly for patients with nocturnal asthma.

Prophylactic therapy is typically provided by anti-inflammatory glucocorticosteroids such as beclomethasone dipropionate, budesonide, fluticasone propionate, mometasone furoate and ciclesonide. Recent therapeutic strategy is aimed at both controlling the symptoms and reducing the inflammation by combinations of a long-acting beta-2 agonist and a glucocorticosteroid.

In any case, it is normal clinical practice to administer combination inhalers twice, sometimes once, daily at a dose related to the severity of asthma as a maintenance therapy and to use a short-acting beta-2 agonist, such as salbutamol or terbutaline, as required to relieve any breakthrough symptoms and acute situations.

These acute situations may increase in frequency and severity requiring additional administration of short-acting beta-2 agonist.

Inhalation has become the primary route of administration in the treatment of asthma. Typical delivery systems for inhalable drugs are: pressurised metered dose inhalers, dry powder inhalers, or nebulizers (ultrasonic, jet, soft-mist etc.).

Complicated therapy with different medications and devices may lead to poor compliance of the patients, so to under-treatment and, in turn, negative impact on their quality of life. This is dramatically evident in the case of long-term management of chronic asthma, in particular with prophylactic treatments, such as with inhaled glucocorticosteroids, which do not give immediate symptom relief.

Therefore, there is a need to simplify present asthma treatment providing a user friendly, efficacious and safe treatment.

In this direction WO 99/64014 proposes the use of a budesonide-formoterol combination for prevention or treatment of an acute condition of asthma.

The present invention proposes an alternative combination featuring an enhanced efficacy and showing an excellent safety profile.

### SUMMARY OF THE INVENTION

The present invention provides the use of a suitable composition for the manufacture of a medicament for the rescue treatment of acute episodes as a complement to the maintenance treatment of asthma.

More specifically the present invention relates to the use of a composition comprising a fixed combination of
a) formoterol, a pharmaceutically acceptable salt or solvate thereof or a solvate of such a salt; and
b) beclomethasone dipropionate;
for the manufacture of a medicament for use in the prevention or treatment of an acute condition of asthma, intermittent asthma and/or episodes in chronic asthma, more preferably in the rescue treatment of asthma, and in particular in case of acute exacerbations during the maintenance, therapy of asthma.

The use of the present composition relates to the use during one or more of the following conditions:
i) an acute condition of asthma, i.e. acute asthma attack, when a patient on an irregular basis can be exposed to an agent i.e. pollen, a virus infection, cold air, exercise, perfumes or any other agent triggering an asthma attack;
ii) an intermittent asthma, i.e. in case of occasional (less than twice/week), brief exacerbations (hours to days), wherein nocturnal exacerbations are less than twice/month;
iii) episodes, i.e. short periods of acute attacks of bronchospasms in chronic asthma.

### DETAILED DESCRIPTION OF THE INVENTION

According to the present invention, a fixed combination of formoterol and beclomethasone dipropionate may be administered in addition to the maintenance treatment of chronic asthma on a regular basis for the treatment of an acute conditions of asthma, an intermittent asthma and/or episodes in chronic asthma.

In fact, in assessing the acute tolerability of high, cumulative doses of the combination formoterol and beclomethasone dipropionate in comparison with formoterol alone or to placebo, we have surprisingly found that formoterol alone caused significantly greater decrease in serum potassium level than the fixed combination or placebo.

Therefore the proposed combination, even when administered in doses largely in excess than the recommended clinical dose, as in case of a rescue treatment of asthma, and in particular in case of acute exacerbations during the maintenance therapy of asthma, is safer than formoterol alone as shown in Example 3.

During the maintenance therapy of asthma with a regular administration of the present composition, i.e. twice daily, in case of exacerbations the symptoms can be counteracted by incrementing dosages of the same composition both permitting to the additional glucocorticosteroid component (beclomethasone dipropionate) coming in as early as possible to suppress the enhanced airway inflammation and to the additional long-acting beta-2 agonist (formoterol) to immediately reduce the bronchial constriction, therefore minimising the risk of too frequent dosing of different inhalers.

According to the present invention the administration of the combination formoterol/beclomethasone dipropionate reduces the severity of exacerbations and minimizes the difficulty of predicting the best dosage regimen of glucocorticosteroid (low dose or high dose) before adding the long-acting beta-2 agonist.

Moreover in certain patients wherein a too low maintenance dose of the combination is administered and the glucocorticosteroid is under the dosage required to give its beneficial anti-inflammatory effects, the as needed use, or rescue use, of the combination according to the invention will re-establish the effective therapeutic dose of the glucocorticosteroid even if used only for a rescue purpose.

The composition of the present invention comprises a fixed combination of formoterol and beclomethasone dipropionate.

Formoterol, (±) N-[2-hydroxy-5-(1-hydroxy-2-((2-(4-methoxyphenyl)-1-methylethyl)amino)-ethyl) phenyl] formamide, particularly in the form of its fumarate salt, is a bronchodilator used in the treatment of inflammatory or obstructive airways diseases, Formoterol can exist in four stereochemical forms. The present invention includes the individual stereoisomers, and in particular the (R, R)-enantiomer, as well as mixtures thereof and preferably its racemic mixture.

Formoterol may be present in the composition as free base or as a pharmaceutically acceptable salt. Pharmaceutically acceptable salt of formoterol include, for example, salts of inorganic acids such as hydrochloric, hydrobromic, sulfuric, phosphoric acids and of organic acids such as maleic, fumaric, tartaric, citric, benzoic, 4-methoxybenzoic, 2- or 4-hydroxybenzoic, 4-chlorobenzoic, p-toluenesulphonic, methanesulphonic, ascorbic, salicylic, acetatic, succinic, lactic, tricarballylic, hydroxy-naphthalene-carboxylic, gluconic, oleic acids.

Formoterol or its salts may be present in the composition in a particular crystalline form. The preferred salt of formoterol is formoterol fumarate, particularly in the form of dihydrate.

Beclomethasone dipropionate, (1-beta, 16beta)-9-chloro-11, 17, 21-trihydroxy-16-methylpregna-1, 4-diene-3, 20-dione 17, 21-dipropionate, is a well known anti-inflammatory glucocorticosteroid, used by inhalation as an antiasthmatic compound.

The molar ratio of formoterol or pharmaceutically acceptable salt thereof to beclomethasone dipropionate in a fixed combination, calculated as formoterol to beclomethasone dipropionate, is generally from 1:1 to 1:500, preferably from 1:1 to 1:100, more preferably from 1:1 to 1:60, even more preferably from 1:3 to 1:30, most preferably from 1:12 to 1:26 and the most preferred ratio is 1;12.8 or 1:25.6.

A recommended maximum daily dose of formoterol in combination with beclomethasone dipropionate for adults is 24 micrograms.

A recommended maximum daily dose of beclomethasone dipropionate in combination with formoterol for adults is 400 micrograms.

These doses are recommended to avoid the exposition of the patient to high levels of the active compounds. However, in the present invention it has been found that it is possible for the patient to administer this mixture as often as needed.

In particular, the daily dose of formoterol for adults, including maintenance therapy, may be as high as 168 micrograms, preferably 100 micrograms, and more preferably 84 micrograms and even more preferably 72 micrograms.

The daily dose of beclomethasone dipropionate for adults, including maintenance therapy, may be as high as 5600, preferably 2800 micrograms, more preferably 2400 micrograms, even more preferably 1400 and most preferably 1200 micrograms.

The dose regimen will depend on the severity of the disease if mild, moderate or severe asthma and on the patient's age, sex, weight etc.

The combination may be administered by inhalation orally or intranasally. The combination is preferably administered by a dry powder inhaler, a pressurized metered dose inhaler, or a nebuliser.

When the combination of the invention is formulated in the form of a dry powder composition to be administered by a dry powder inhaler, for example a single or a multi dose inhaler, both the active ingredients are in the form of micronized particles, preferably with a particle size of less than 10 micron,

The composition may comprise one or more suitable diluents or carriers such as lactose, dextran, mannitol or glucose. Preferably lactose is used, more preferably alpha-lactose monohydrate. Both the active ingredients of the combination of the invention and the diluent carrier may be in a micronized form. Their mixture can optionally be subjected to agglomeration and/or spheronization.

Otherwise a coarse carrier/diluent may be added to the composition comprising the active ingredients of the combination and optionally a diluent/carrier in the form of micronized particles, to form an ordered mixture. Said ordered mixture may optionally contain an additive to promote the release of the active ingredients.

Suitable additives are substances with anti-adherent, glidant or lubricant properties. Magnesium stearate is a particularly preferred additive.

When the combination of the invention is formulated in the form of a pressurized metered dose inhaler, the active ingredients may be both in micronized form suspended or more preferably both completely dissolved in a liquid propellant mixture. Alternatively one of the two active ingredients may be suspended and the other completely dissolved in the liquid propellant mixture.

The propellants which can be used include chlorofluorocarbons, hydrocarbons or hydrofluorocarbons. Especially preferred propellants are HFA 134a (1,1,1,2-tetrafluoroethane), HFA 227 (1,1,1,2,3,3,3-heptafluropropane) or their mixtures. The active ingredients propellant mixtures are optionally used in combination with one or more other propellants and/or one or more additives such as cosolvents, for example ethanol, surfactants and/or one or more lubricant, antioxidant, stabilizing and or preserving agents such as an aqueous mineral acid and preferably 1M hydrochloric acid.

When the combination of the invention is formulated in the form of a pressurized metered dose inhaler, particularly preferred are solution formulations wherein the two active ingredients of the composition are dissolved in the propellant mixture. More preferably the propellant mixture comprises and/or consists of hydroflurocarbons, such as HFA 134a, HFA 227 or their mixtures, a cosolvent, such as ethanol and an aqueous mineral acid, such as 1M hydrochloric acid, as a stabilizing agent.

Particularly preferred are HFA solution formulations (as the one of the following Examples 1, 2 and 4) which upon actuation of the pressurized metered dose inhaler, on evaporation of the propellant mixture, feature an average particle size of the two active ingredients equal or below 1.1 micrometer. These formulations are also referred herewith as extrafine formulations. Extrafine formulations assure a co-deposition in the lung areas of both the active ingredients particles. This co-deposition results in enhanced therapeutic bronchodilator effects and in a safer medicament.

In fact, due to its optimized particle distribution and increased deposition in the peripheral airways, the solution formulation combination of the present invention allows a reduction in the glucocorticosteroid dose and a consequent reduction of its systemic exposure with respect to the marketed budesonide - formoterol dry powder inhaler formulations as described in WO 99/64014 (Examples 3-4). In fact according to GINA (Global Initiative for Asthma) international guidelines, Workshop Report Updated 2003, daily doses of 400 micrograms of beclomethasone dipropionate HFA (extrafine pressurized solution formulation) and 800 micrograms of budesonide dry powder inhaler formulation are equivalent.

Therefore the availability of an extrafine HFA solution formulation of the combination of the present invention may further improve the risk/benefit ratio over the prior art formulation in case of multiple administrations of the combination such as in the rescue treatment of asthma, and in particular in case of acute exacerbations during the maintenance therapy of asthma.

When the combination of the invention is formulated in the form of a nebuliser the active ingredients may be both in micronized form suspended, dissolved or alternatively one is dissolved and the other is suspended to give a nebulised aqueous or hydroalcoholic suspension or solution, available either as a unit dose or multi-dose formulation, with or without suitable pH or tonicity adjustment and optional addition of stabilizing and or preserving agent.

Other features of the invention will become apparent in the course of the following descriptions of exemplary embodiments which are given for illustration of the invention and are not intended to be limiting thereof

In the following examples micronization is carried out in a conventional manner such that the particle size range for each component is suitable for administration by inhalation.

### EXAMPLE 1

A pressurized metered dose inhaler solution formulation of the combination formoterol fumarate + beclomethasone dipropionate contained formoterol fumarate dihydrate 6 micrograms/dose (0.010% w/w based on the total weight of the formulation), beclomethasone dipropionate 100 micrograms/dose (0.172% w/w), ethanol 12% w/w as cosolvent and hydrochloric acid (1M) 0,024% w/w as stabilizing agent and HFA 134a to 100% as the propellant. The formulation was packed in aluminium cans fitted with 50 microliters valves.

### EXAMPLE 2

A pressurized metered dose inhaler solution formulation of the combination formoterol fumarate + beclomethasone dipropionate contained formoterol fumarate dihydrate 6 micrograms/dose (0.008% w/w based on the total weight of the formulation), beclomethasone dipropionate 200 micrograms/dose (0.271% w/w), ethanol 12% w/w as cosolvent and hydrochloric acid (1M) 0.019% w/w as stabilizing agent and HFA 134a to 100% as the propellant. The formulation was packed in aluminium cans fitted with 63 microliters valves.

### EXAMPLE 3

A dry powder inhaler formulation of the combination formoterol fumarate + beclomethasone dipropionate contained micronized formoterol fumarate dihydrate 6 micrograms/dose (0,06% w/w based on the total weight of the formulation), micronized beclomethasone dipropionate 100 micrograms/dose (1% w/w), 989 micrograms/dose of a preblend mixture (9,89% w/w) constituted of micronized lactose and magnesium stearate 98:2 w/w), 8904 micrograms/dose of coarse alpha-lactose monohydrate having a particle size comprised between 212 and 355 micron (89,04% w/w).

### EXAMPLE 4

A study was performed to evaluate the tolerability of high, cumulative doses of the fixed combination formoterol/beclomethasone dipropionate or formoterol compared to placebo when administered in asthmatic patients on regular treatment with the combination at the maximum recommended daily dose (6 micrograms of formoterol / 100 micrograms beclomethasone dipropionate, 2 puffs bid.: corresponding to a total daily dose of 24 micrograms of formoterol and of 400 micrograms of beclomethasone dipropionate).

The study was a double blind clinical comparison study of ten puffs of the pressurized metered dose inhaler solution formulation of the combination of Example 1 (formoterol 6 micrograms + beclomethasone dipropionate 100 micrograms) or formoterol (6 micrograms) or placebo during the maintenance treatment of asthma with 2 puffs bid. of the combination formoterol/beclomethasone dipropionate of Example 1.

Cumulative doses (10 puffs) were administered on 3 separated days in addition to the morning dose maintenance (2 puffs). Primary endpoint was serum potassium, assessed over a 12 hours period after the cumulative doses. In addition the effects of the treatment on the ECG (electrocardiogram), QTc (QT interval corrected of the heart's electrical cycle), blood pressure and heart rate were evaluated at regular intervals over a 12 hours period after dosing. Plasma lactate and glucose were determined over 3 hours after dosing.

Formoterol alone caused significantly greater decrease in serum potassium level than the fixed combination or placebo. While no significant differences in serum potassium parameters were observed between the fixed combination and placebo.

QTc, plasma lactate and the other vital signs values observed with the combination were not statistically different from those with formoterol alone.

In conclusion the administration of high cumulative doses of the combination formoterol/beclomethasone dipropionate in asthmatic patients on maintenance treatment with the combination do not significantly reduce their serum potassium levels differently from formoterol alone. Therefore the combination even when administered in doses largely in excess than the recommended clinical dose, such as in case of the rescue treatment of asthma, and in particular in case of acute exacerbations during the maintenance therapy of asthma, is safer than formoterol in asthmatic patients.

## Claims

1. The use of a composition comprising a fixed combination of
a) formoterol, a pharmaceutically acceptable salt or solvate thereof or a solvate of such a salt and
b) beclomethasone dipropionate
for the manufacture of a medicament for use in the prevention or treatment of an acute condition of asthma, intermittent asthma and/or episodes in chronic asthma.

2. Use of a composition comprising a fixed combination of
a) formoterol, a pharmaceutically acceptable salt or solvate thereof or a solvate of such a salt and
b) beclomethasone dipropionate for the manufacture of a medicament for use in the rescue treatment of asthma, and in particular in case of acute exacerbations during the maintenance therapy of asthma.

3. Use according to any one of claims 1 and 2, wherein the molar ratio of (a) to (b), calculated as formoterol to beclomethasone dipropionate, is from 1:1 to 1:500, preferably from 1:1 to 1:100, more preferably from 1:1 to 1:60.

4. Use according to claim 3, wherein the molar ratio of (a) to (b), calculated as formoterol to beclomethasone dipropionate, is from 1:3 to 1:30, preferably from 1:12 to 1:26.

5. Use according to claim 4, wherein the molar ratio of (a) to (b), calculated as formoterol to beclomethasone dipropionate, is 1:12.8.

6. Use according to claim 4, wherein the molar ratio of (a) to (b), calculated as formoterol to beclomethasone dipropionate, is 1:25.6.

7. Use according to any one of claims 1 to 6 wherein the first active ingredient is formoterol fumarate dihydrate.

8. Use according to any one of claims 1 to 6 wherein the first active ingredient is the R,R-enantiomer of formoterol or pharmaceutically acceptable salt or solvate thereof or a solvate of such a salt.

9. Use according to any previous claim wherein the daily dose of formoterol for adults, including maintenance therapy, is as high as 168 micrograms, preferably 100 micrograms, and more preferably 84 micrograms and even more preferably 72 micrograms.

10. Use according to any previous claim wherein the daily dose of beclomethasone dipropionate for adults, including maintenance therapy, is as high as 5600, preferably 2800 micrograms, more preferably 2400 micrograms, even more preferably 1400 and most preferably 1200 micrograms.

11. Use according to any previous claim, wherein the composition is administered by inhalation orally or intranasally.

12. Use according to claim 10, wherein the combination is administered by a dry powder inhaler, a pressurized metered dose inhaler, or a nebuliser.

13. Use according to claim 11, wherein the combination is formulated in form of a dry powder composition and may comprise one or more suitable diluents or carriers such as lactose, dextran, mannitol or glucose and preferably alpha-lactose monohydrate.

14. Use according to claim 12, wherein both the active ingredients (a) and (b) of the combination of the invention and the diluent carrier may be in a micronized form.

15. Use according to claim 13, wherein a coarse carrier/diluent may be added to the composition to form an ordered mixture

16. Use according to claim 14, wherein said ordered mixture may optionally contain an additive to promote the release of the active ingredients selected from substances with anti-adherent, glidant or lubricant properties such as magnesium stearate.

17. Use according to claim 11, wherein the combination is formulated in the form of a pressurized metered dose inhaler and wherein both the active ingredients (a) and (b) in micronized form are suspended in a liquid propellant mixture.

18. Use according to claim 11, wherein the combination is formulated in the form of a pressurized metered dose inhaler and wherein one of the two active ingredient is suspended and the other is completely dissolved in a liquid propellant mixture.

19. Use according to claim 11, wherein the combination is formulated in the form of a pressurized metered dose inhaler wherein both the active ingredients (a) and (b) are completely dissolved in a liquid propellant mixture.

20. Use according to claim 18, wherein the liquid propellant mixture comprises HFA 134a (1,1,1,2-tetrafluoroethane), HFA 227 (1,1,1,2,3,3,3-heptafluropropane) or their mixtures, optionally in combination with one or more cosolvent, surfactant, lubricant, antioxidant, stabilizing and or preserving agent.

21. Use according to claim 19, wherein the liquid propellant mixture comprises HFA 134a (1,1,1,2-tetrafluoroethane), ethanol as a cosolvent and an aqueous mineral acid as stabilising agent.

22. Use according to claim 20, wherein the aqueous mineral acid is 1M hydrochloric acid.

23. Use according to any one of claims 18 to 21 which upon actuation of the pressurized metered dose inhaler, on evaporation of the propellant mixture, features an average particle size of the two active ingredients equal or below 1.1 micrometer.

24. Use according to claim 11, wherein the combination is formulated in the form of a nebulizer either as a unit dose or multidose formulation.

25. Use according to claim 23, wherein both the active ingredients (a) and (b), in micronized form are suspended to give a nebulized aqueous or hydroalcoholic suspension with or without suitable pH or tonicity adjustment and optional addition of stabilizing and or preserving agent.

26. Use according to claim 23, wherein both the active ingredients (a) and (b) are dissolved to give a nebulized aqueous or hydroalcoholic solution with or without suitable pH or tonicity adjustment and optional addition of stabilizing and or preserving agent.

27. Use according to claim 23, wherein one of the two active ingredient is suspended and the other is completely dissolved to give a nebulized aqueous or hydroalcoholic suspension with or without suitable pH or tonicity adjustment and optional addition of stabilizing and or preserving agent.
